# EUROPEAN PATENT APPLICATION

(11) **EP 1 810 980 A1**
(43) Date of publication of application: **25.07.2007**
(21) Application number: 05805371.1
(22) Date of filing: 27.10.2005
(51) Int. Cl.: C07K 16/28, C12N 15/00, C12P 21/08, A61K 39/395, A61P 29/00

(54) **INTERLEUKIN-6 INHIBITORS**

(30) Priority: 28.10.2004 US 623018 P
(71) Applicant: OSAKA UNIVERSITY, Suita-shi, Osaka 565-0871 (JP); THE UAB RESEARCH FOUNDATION, Birmingham, AL 35294-0110 (US); CHUGAI SEIYAKU KABUSHIKI KAISHA, Tokyo, 115-8543 (JP)
(72) Inventor: CURIEL, David T., Birmingham, Alabama 35222 (US); PEREBOEV, Alexander, Birmingham, Alabama 35216 (US); ADACHI, Yasuo, Laboratory of Immune Regulation, Suita-shi, Osaka 5650871 (JP); KISHIMOTO, Tadamitsu, Tondabayashi-shi, Osaka 5840021 (JP); NISHIMOTO, Norihiro, Minoh-shi, Osaka 5620011 (JP)
(74) Representative: Vossius & Partner
(86) International application number: PCT/JP2005/019820
(87) International publication number: WO 2006/046661

(57) **Abstract**

The present invention provides modified antibodies comprising scFv and Fc. In the modified antibodies of this invention, the L-chain and H-chain variable regions are expressed in single chains as scFv. The modified antibodies of this invention are expressed as single-chain polypeptides further comprising Fc regions. Vectors with a simple configuration can be used to reproduce structures mimicking native IgGs. In particular, polynucleotides encoding the modified antibodies of the present invention can be utilized as gene therapy vectors for *in vivo* expression of antibody molecules useful as IL-6 receptor inhibitors.

## Description

### Technical Field

The present invention relates to modified antibodies.

### Background Art

Interleukin-6 (IL-6) is a cytokine also referred to as B cell stimulating factor 2 (BSF2) or interferon β2. IL-6 was discovered as a differentiation factor involved in the activation of B lymphoid cells (Non-Patent Document 1), and was later found to be a multifunctional cytokine affecting the functions of a variety of cells (Non-Patent Document 2). IL-6 has been reported to induce maturation of T lymphoid cells (Non-Patent Document 3).

IL-6 transmits its biological activity via two types of proteins on cells. One of them is the IL-6 receptor, a ligand-binding protein having a molecular weight of approximately 80 kD and to which IL-6 binds (Non-Patent Documents 4 and 5). In addition to the membrane-bound form that is expressed on the cell membrane and penetrates through the cell membrane, the IL-6 receptor is also present as a soluble IL-6 receptor, which is mainly composed of its extracellular region.

Patent Document 1 describes the various forms of anti-IL-6R antibodies, for example, humanized anti-IL-6R antibodies, chimeric anti-IL-6R antibodies and such-. Patent Document 2 describes therapeutic agents for chronic rheumatoid arthritis and synovial cell proliferation inhibitors comprising an IL-6 antagonist, such as an anti-IL-6R antibody, as the active ingredient. Patent Document 3 describes treatments for diseases caused by IL-6 production, such as plasmacytosis, hyperimmunoglobulinemia, anemia, nephritis, cachexia, rheumatism, Castleman's disease, and mesangioproliferative nephritis. Patent Document 4 describes preventive and therapeutic agents for sensitized T cell-mediated diseases, such as multiple sclerosis, uveitis, chronic thyroiditis, delayed hypersensitivity, contact dermatitis, and atopic dermatitis, which comprise an anti-IL-6R antibody as the active ingredient.

Patent Document 4 describes therapeutic agents for systemic lupus erythematosus, where the agents comprise an anti-IL-6R antibody as the active ingredient. Patent Document 5 describes therapeutic agents for Crohn's disease, where the agents comprise an anti-IL-6R antibody as the active ingredient. Patent Document 6 describes therapeutic agents for pancreatitis, where the agents comprise an anti-IL-6R antibody as the active ingredient. Patent Document 7 describes therapeutic agents for psoriasis, where the agents comprise an anti-IL-6R antibody as the active ingredient. Patent Document 8 describes therapeutic agents for childhood chronic arthritis, where the agents comprise an anti-IL-6R antibody as the active ingredient.
[Patent Document 1] WO92/19759
[Patent Document 2] WO96/11020
[Patent Document 3] WO96/12503
[Patent Document 4] WO98/42377
[Patent Document 5] WO99/47170
[Patent Document 6] WO00/10607
[Patent Document 7] WO02/34292
[Patent Document 8] WO02/080969
[Non-Patent Document 1] Hirano, T. et al., Nature (1986) 324, 73-76
[Non-Patent Document 2] Akira, S. et al., Adv. in Immunology (1993) 54, 1-78
[Non-Patent Document 3] Lotz, M. et al., J. Exp. Med. (1988) 167, 1253-1258
[Non-Patent Document 4] Taga, T. et al., J. Exp. Med. (1987) 166, 967-981
[Non-Patent Document 5] Yamasaki, K. et al., Science (1988) 241, 825-828

### Disclosure of the Invention

### Problems to be Solved by the Invention

Clinical studies have shown that anti-IL-6 receptor antibodies are effective for treating rheumatoid arthritis (chronic rheumatoid arthritis) and Castleman's disease. The inventors of this application have submitted a patent application for therapeutic agents for mesothelioma such as pleural mesothelioma, where the agents comprise an anti-IL-6R antibody as the active ingredient (WO2005/037315). Furthermore, the inventors of this application have submitted a patent application for therapeutic agents for vasculitis such as polyarteritis nodosa, aortitis syndrome, and vasculitis associated with immune disorders, where the agents comprise an anti-IL-6R antibody as the active ingredient (WO2005/061000). Herein, since the cost of treatments using antibody pharmaceuticals is generally expensive, the development of vectors for efficiently expressing anti-IL-6 receptor antibodies (IL-6 inhibitors) becomes extremely important in terms of reducing costs.

However, since the H chain and L chain of an antibody are coded on two separate genes, incorporating the genes in their original form into viral vectors or such and introducing the genes into target cells would be difficult to express and secrete effective therapeutic agents.

An objective of the present invention is to provide modified antibodies that can be efficiently expressed as anti-IL-6 receptor antibodies that are useful as IL-6 inhibitors, and to provide vectors for expressing these antibodies, as well as to provide production methods and uses thereof.

### Means to Solve the Problems

The present inventors modified the structure of an antibody to enable efficient expression of anti-IL-6 receptor antibodies. The present inventors thus discovered that as a structure that has the activity of inhibiting anti-IL-6 receptor and that can be produced efficiently, a useful structure is one in which an scFv is connected to a human immunoglobulin IgG Fc region, wherein the scFv comprises an anti-IL-6 receptor antibody H-chain and L-chain V region, and they thus completed the present invention. More specifically, the present invention provides the following modified antibodies, vectors that express them, and production methods and uses thereof.
[1] a modified antibody with an scFv connected to a human immunoglobulin IgG Fc region, wherein said scFv comprises an anti-IL6 receptor antibody H-chain and L-chain V region, and CDRs comprising of the following amino acid sequences incorporated into a human immunoglobulin framework:
   CDR1 in H-chain V region: SDHAWS (SEQ ID NO: 3)
   CDR2 in H-chain V region: YISYSGITTYNPSLKS (SEQ ID NO: 4)
   CDR3 in H-chain V region: SLARTTAMDY (SEQ ID NO: 5)
   CDR1 in L-chain V region: RASQDISSYLN (SEQ ID NO: 6)
   CDR2 in L-chain V region: YTSRLHS (SEQ ID NO: 7)
   CDR3 in L-chain V region: QQGNTLPYT (SEQ ID NO: 8);
[2] the modified antibody of [1], wherein the H-chain V region consists of the amino acid sequence of QVQLQESGPGLVRPSQTLSLTCTVSGYSITSDHAWSWVRQPPGRGLEWIG YIS-YSGITTYNPSLKS RVTMLRDTSKNQFSLRLSSVTAADTAVYYCAR SLARTTAMDY WGQGSLVTVS (SEQ ID NO: 1);
[3] the modified antibody of [1], wherein the L-chain V region consists of the amino acid sequence of DIQMTQSPSSLSASVGDRVTITC RASQDISSYLN WYQQKPGKAPKLLIY YTSRLHS GVPSRFSGSGSGTDFTFTISSLQPEDIATYYC QQGNTLPYT FGQGTKVEIKR (SEQ ID NO: 2);
[4] the modified antibody of [1], wherein the scFv is connected to the human immunoglobulin IgG Fc region at the C terminus of the L-chain V region;
[5] the modified antibody of [4], wherein the scFv comprises an H-chain V region, a linker, and an L-chain V region connected in that order from the N terminus;
[6] The modified antibody of [5], wherein the amino acid sequence of the linker is GGGGSGGRASGGGGSGGGGS (SEQ ID NO: 9);
[7] the modified antibody of [1], which is a dimer connected via an SS bond;
[8] a pharmaceutical composition comprising the modified antibody of any one of [1] to [7] and a pharmaceutically acceptable carrier;
[9] a method for manufacturing a pharmaceutical composition comprising the step of combining the modified antibody of any one of [1] to [7] and a pharmaceutically acceptable carrier;
[10] a polynucleotide encoding a modified antibody which comprises an scFv connected to a human immunoglobulin IgG Fc region, wherein said scFv comprises an anti-IL-6 receptor antibody H-chain and L-chain V region, and CDRs comprising of the following amino acid sequences incorporated into a human immunoglobulin framework:
   CDR1 in H-chain V region: SDHAWS (SEQ ID NO: 3)
   CDR2 in H-chain V region: YISYSGITTYNPSLKS (SEQ ID NO: 4)
   CDR3 in H-chain V region: SLARTTAMDY (SEQ ID NO: 5)
   CDR1 in L-chain V region: RASQDISSYLN (SEQ ID NO: 6)
   CDR2 in L-chain V region: YTSRLHS (SEQ ID NO: 7)
   CDR3 in L-chain V region: QQGNTLPYT (SEQ ID NO: 8);
[11] the polynucleotide of [10], wherein the H-chain V region consists of the amino acid sequence of QVQLQESGPGLVRPSQTLSLTCTVSGYSIT SDHAWS WVRQPPGRGLEWIG YIS-YSGITTYNPSLKS RVTMLRDTSKNQFSLRLSSVTAADTAVYYCAR SLARTTAMDY WGQGSLVTVS (SEQ ID NO: 1);
[12] the polynucleotide of [10], wherein the L-chain V region consists of the amino acid sequence of DIQMTQSPSSLSASVGDRVTITC RASQDISSYLN WYQQKPGKAPKLLIY YTSRLHS GVPSRFSGSGSGTDFTFTISSLQPEDIATYYC QQGNTLPYT FGQGTKVEIKR (SEQ ID NO: 2);
[13] the polynucleotide of [10], wherein the scFv is connected to a human immunoglobulin IgG Fc region at the C terminus of an L-chain V region;
[14] the polynucleotide of [13], wherein the scFv comprises an H-chain V region, a linker, and an L-chain V region connected in that order from the N terminus;
[15] the polynucleotide of [14], wherein the amino acid sequence of the linker is GGGGSGGRASGGGGSGGGGS (SEQ ID NO: 9);
[16] a vector comprising the polynucleotide of any one of [10] to [15]
[17] a transformant harboring the polynucleotide of any one of [10] to [15] or a vector comprising said polynucleotide in an expressible manner;
[18] the transformant of [17], wherein the transformant is derived from a human cell;
[19] a method for manufacturing a modified antibody comprising the steps of culturing the transformant of [17] and recovering a modified antibody accumulated in culture;
[20] an IL-6 receptor inhibitor comprising as an active ingredient a component selected from the group consisting of the modified antibody of any one of [1] to [7]; the polynucleotide of any one of [10] to [15]; a vector carrying the polynucleotide of any one of [10] to [15] in an expressible manner; and a cell producing the modified antibody of any one of [1] to [7];
[21] a method for inhibiting IL-6 receptor comprising the step of administering any components selected from the group consisting of the modified antibody of any one of [1] to [7]; the polynucleotide of any one of [10] to [15]; a vector carrying the polynucleotide of any one of [10] to [15] in an expressible manner; and a cell producing the modified antibody of any one of [1] to [7];
[22] a gene therapy vector comprising the polynucleotide of any one of [10] to [15] such that it can be expressed in a human cell;
[23] a method for treating a disease caused by an action of IL-6, wherein the method comprises the steps of:
   (1) introducing into a cell a vector carrying the polynucleotide of any one of [10] to [15] such that it can be expressed in a human cell; and
   (2) administering the vector-introduced cell to a patient;
[24] the method of [23], wherein the cell is obtained from a patient to be treated;
[25] the method of [24], wherein the cell is a peripheral blood lymphocyte; and
[26] a therapeutic kit for a disease caused by an action of IL-6, wherein the kit comprises the following elements:
   (1) a vector carrying the polynucleotide of any one of [10] to [15] such that it can be expressed in a human cell;
   (2) a reagent for vector introduction; and
   (3) a reagent for peripheral blood lymphocyte isolation.

### Brief Description of the Drawings

Fig. 1 shows a pShuttle plasmid incorporating an SV40 late polyA signal.
Fig. 2 shows subcloning of human immunoglobulin IgG1 into the BamHI site of pBluescript II.
Fig. 3 shows the IL-6 inhibitor-(modified antibody)-expressing shuttle vector.
Fig. 4 shows a candidate IL-6 signal blocker derived from a humanized anti-IL-6 receptor humanized antibody.
Fig. 5 is a graph showing the affinity of a modified antibody of the present invention in comparison to that of an anti-IL-6 receptor humanized antibody. The horizontal axis shows the concentration of the modified antibody of the present invention, the anti-IL-6 receptor humanized antibody, and the control IgG that were reacted.
Fig. 6 is a graph showing the ability of the modified antibody of the present invention to inhibit IL-6 receptor binding. The horizontal axis shows the concentrations of the modified antibody of the present invention and anti-IL-6 receptor humanized antibody that were reacted.
Fig. 7 is a graph showing the effect of a modified antibody of the present invention in suppressing KT-3 cell growth. The horizontal axis shows the concentration of the modified antibody of the present invention, the anti-IL-6 receptor humanized antibody, and the control IgG that were reacted.
Fig. 8 is a graph showing the effect of a modified antibody of the present invention in suppressing the enhancement of VEGF production caused by IL-6 and sIL-6R stimulus in malignant pleural mesothelioma. The vertical axis indicates VEGF concentration.
Fig. 9 shows the position of each of the elements constituting the construct, and the nucleotide sequences that connect the elements.
Fig. 10 shows SEQ ID NO: 10 and the amino acid sequence (SEQ ID NO: 11) of the scFv encoded by SEQ ID NO: 10. The italicized regions indicate linker regions.

### Best Mode for Carrying Out the Invention

An objective of the present invention is to modify an anti-IL-6 receptor antibody into a protein encoded by a single gene that is suitable for gene transfer using an adenovirus vector or such, and to provide IL-6 inhibitors suitable for gene therapy. Further, the present invention provides anti-IL-6 receptor antibodies encoded by single genes that enable more efficient production of anti-IL-6 receptor antibodies through expression of antibody H chains and L chains in equal amounts. That is, the present invention relates to modified antibodies comprising an scFv and a human immunoglobulin IgG Fc, in which the scFv comprises anti-IL-6 receptor antibody H-chain and L-chain V regions, and CDRs comprising each of the following amino acid sequences incorporated into a human immunoglobulin framework:
CDR1 in H-chain V region: SDHAWS (SEQ ID NO: 3)
CDR2 in H-chain V region: YISYSGITTYNPSLKS (SEQ ID NO: 4)
CDR3 in H-chain V region: SLARTTAMDY (SEQ ID NO: 5)
CDR1 in L-chain V region: RASQDISSYLN (SEQ ID NO: 6)
CDR2 in L-chain V region: YTSRLHS (SEQ ID NO: 7)
CDR3 in L-chain V region: QQGNTLPYT (SEQ ID NO: 8)

The modified antibodies of the present invention are constructed by connecting an scFv comprising an anti-IL-6 receptor antibody H-chain and L-chain V region to a human immunoglobulin IgG Fc region. An scFv is also referred to as a single chain variable fragment. Generally, an scFv or scFv antibody fragment includes antibody VH and VL regions. These regions are present in a single polypeptide chain (Huston, J. S. et al., Proc. Natl. Acad. Sci. U.S.A. (1988) 85, 5879-5883). Generally, scFv polypeptides further comprise polypeptide linkers between the V_{H} and V_{L} regions. Depending on the linker, an scFv can form a structure necessary for antigen binding (for a review on scFv, see Pluckthun "The Pharmacology of Monoclonal Antibodies" Vol.113 (Rosenburg and Moore ed (Springer Verlag, New York) pp.269-315, 1994).

In the present invention, the amino acid sequences of linkers constituting scFv are not particularly limited, so long as its binding characteristics as an antibody are maintained when the H-chain and L-chain variable regions are linked to form single chains. For example, (G₄S)(GGRAS)(G₄S)₂ or (G₄S)₃ and the like are known to serve as such linkers.

The order in which the H-chain and L-chain V regions are connected in scFv is not particularly limited so long as its binding characteristics as an antibody is maintained. Specifically, scFv comprising an [H-chain V region]-[linker]-[L-chain V region] connected in that order from the N terminus are preferred as the scFv of the present invention. More specifically, preferred modified antibodies of the present invention are composed of single-chain polypeptides having the following structure:
H₂N-[H-chain V region]-[linker]-[L-chain V region]-[Fc]-COOH

In the present invention, an Fc of an IgG is not limited so long as it is an Fc derived from a human IgG. More specifically, an Fc of any one of Cγ1 to Cγ4 may be used as the Fc constituting the modified antibodies of the present invention. Generally, the Fc region of human IgG is composed of approximately 330 amino acids on the C terminal end of IgG. Fc constituting a modified antibody of the present invention may have amino acid sequence substitutions, deletions, insertions, or additions within a range that allows the modified antibody to maintain IL-6 receptor agonist activity.

For example, minibodies comprising CH3 as a Fc are known to be able maintain the function of the Fc region (Hu, S. et al. Cancer Res 56: 3055-3061, 1996). Minibodies have a structure in which CH3 is connected to an scFv. That is, immunoglobulin molecules that lack CH1 and CH2 and comprise only scFv and CH3 are included in the present invention. Alternatively, immunoglobulins comprising scFv and CH3 as essential structures and additionally comprising any region selected from a CH1, hinge, and CH2, or a partial sequence thereof, are also included in the modified antibodies of the present invention.

More specifically, the present invention provides single-chain polypeptides comprising the following component elements (1) to (4), positioned from the N terminus to the C terminus, and polynucleotides encoding such polypeptides:
(1) [H-chain V region];
(2) [Linker];
(3) [L-chain V region]; and
(4) [Fc] or [an Fc fragment comprising an amino acid sequence that constitutes CH3].

Herein, an H-chain V region is an H-chain V region of an anti-IL-6 receptor antibody in which CDR1 to CDR3, comprising the amino acid sequences of SEQ ID NOs: 3-4 respectively, are incorporated into a human immunoglobulin framework. An L-chain V region is an L-chain V region of an anti-IL-6 receptor antibody in which CDR1 to CDR3, comprising the amino acid sequences of SEQ ID NOs: 6-8 respectively, are incorporated into a human immunoglobulin framework.

The modified antibodies of the present invention comprise scFv and Fc. Any Fc derived from a human immunoglobulin may be used in the present invention. Preferred Fc are human IgG-derived Fc. More specifically, for example, Fc of IgG1 can be used as Fc constituting the modified antibodies of the present invention. Methods for isolating Fc-encoding DNAs are known. Alternatively, Fc genes that have already been cloned can be used in the present invention. Specifically, an Fc portion of an H-chain gene of an anti-IL-6 receptor humanized antibody of Patent Document 1, a pdCs-Fc-X expression vector (Protein Engineering vol. 11. pp495-500, 1998), a soluble Fc fusion protein gene (Cancer Res. Vol 60: 2167-2177, 2000), and such can be used as an Fc gene of the present invention.

The modified antibodies of the present invention are preferably homodimers in which two molecules of single-chain polypeptides are connected by SS bonds. The component unit of natural immunoglobulins is ordinarily a heterodimer in which the H chain and L chain are crosslinked by SS bonds. Such heterodimers correspond to the single-chain polypeptides that constitute the above-mentioned modified antibodies of the present invention. IgG carries two such component units. Heterodimers are also crosslinked by SS bonds. Therefore, the structure of natural IgGs can be mimicked in the modified antibodies of the present invention by dimerizing their component unit which is the single-chain polypeptides, by crosslinking them with SS bonds.

In the present invention, SS bonds for dimerization can be constructed by crosslinking any cysteine (C) of a single chain polypeptide. Cysteines preferred for crosslinking are cysteines positioned at the hinge region. More specifically, homodimers in which the cysteines positioned between CH1 and CH2 are crosslinked by an SS bond are preferred as the modified antibodies of the present invention. Polypeptides can be crosslinked using SS bonds by placing them under oxidative conditions. Alternatively, for modified antibodies lacking a hinge, dimerization can also be accomplished through hydrophobic interaction between CH3s.

In natural IgGs, cysteines (C) present in the hinge region are used to construct heterodimers. However, in the present invention, the variable regions are composed of single-chain scFv, and do not require cysteine (C) to bond to VL. Therefore, cysteines (C) positioned at the hinge can be substituted with other amino acids. For example, in the Examples, a cysteine (C) at the hinge region is substituted with a serine (S).

In the present invention, the variable regions of an anti-IL-6 receptor antibody that constitutes an scFv have structures in which the complementarity determining regions (CDRs) in the variable regions (V regions) constituting the heavy chain (H chain) and light chain (L chain) of a mouse monoclonal antibody (PM-1) against the IL-6 receptor are substituted with the corresponding CDR regions of the human antibody V regions. The amino acid sequences of the CDRs of the L-chain V region of the aforementioned mouse anti-IL-6 receptor antibodies are shown as CDR1, CDR2, and CDR3 in row V_{L}PM-1 of Table 2 in Patent Document 1. The amino acid sequences of the CDRs of the H-chain V region of the aforementioned mouse anti-IL-6 receptor antibodies are shown as CDR1, CDR2, and CDR3 in row V_{H}PM-1 of Table 3 in Patent Document 1.

In general, the characteristics of an antibody in binding to an antigen are determined by complementarity determining regions (CDRs). Therefore, by transplanting the above-mentioned CDRs in to any framework, the antigen binding characteristics of an aforementioned mouse monoclonal antibody PM-1 can be reconstructed. For example, CDRs derived from mouse immunoglobulins can be transplanted to a human immunoglobulin framework. Immunoglobulins reconstructed based on such strategies are called reshaped human antibodies.

By comparing with the consensus sequences for the different subgroups of human V regions as defined by E. A. Kabat et al. ((1987) Sequences of Proteins of Immunological Interest, Fourth Edition, U.S. Department of Health and Human Services, U.S. Government Printing Office), regions corresponding to CDRs and FRs from within amino acid sequences constituting the immunoglobulin variable regions can be identified.

Reshaped human antibodies are modified antibodies also known as humanized antibodies. Reshaped human antibodies are constructed by transplanting CDRs of antibodies derived from immunized animals in to the CDR regions of human immunoglobulins. Common genetic engineering techniques for this are also known.

Specifically, CDRs can be substituted using primers that comprise nucleotide sequences encoding mouse antibody CDRs, and that also comprise, at their 3' end, nucleotide sequences complementary to human antibody framework regions (FRs). For each individual mouse CDR, a synthetic primer connected to a nucleotide sequence of a human FR is combined with a primer that anneals to a neighboring FR, and these are used for PCR. When PCR is performed with such a primer set using a human variable region gene as the template, the amplified DNA comprises as the CDR a mouse nucleotide sequence to which is bound a human-derived FR nucleotide sequence. The amplification products of each of the CDRs are designed such that their ends overlap. Finally, all of the respective amplification products which contain three CDRs are mixed and PCR is carried out. The overlapped regions of each of the amplification products anneal to each other and function as primers. As a result, full-length sequences of variable regions in which the CDRs are substituted with mouse-derived nucleotide sequences can be obtained. (See European Patent Application No. EP239400 and International Patent Application No. WO96/02576.) Those human antibody FRs connected via CDRs that are selected are those in which the CDRs form good antigen binding regions.

The human immunoglobulin frameworks of the present invention are not limited. For example, the framework regions (FRs) of an L-chain V region of an aforementioned humanized IL-6 receptor antibody are preferably those derived from human antibody REI. The amino acid sequences of FRs derived from human antibody REI are shown by FR1, FR2, FR3, and FR4 in row REI of Table 2 in Patent Document 1.

The framework regions (FR) of an H-chain V region of an aforementioned humanized IL-6 receptor antibody are preferably those derived from human antibody NEW. The amino acid sequences of the FRs of human antibody NEW are shown by FR1, FR2, FR3, and FR4 in row NEW of Table 3 in Patent Document 1.

Within a humanized V chain composed of human antibody FRs and mouse antibody CDRs, the FR regions can undergo various modifications to improve antigen binding and neutralization activities. For example, it is known that amino acids in the framework regions of antibody variable regions can be substituted such that a suitable antigen binding site will be formed by the complementarity determining regions of the reshaped human antibody (Sato, K. et al., Cancer Res. (1993) 53, 851-856). For the humanized IL-6 receptor antibody used in the present application, RV_{L}a (version a) in Table 2 of Patent Document 1 is preferred for an L-chain V region, and RV_{H}f (version f) in Table 3 of Patent Document 1 is preferred for an H-chain V region. The amino acid sequences of preferred humanized V chains of the present invention are indicated below. In the sequences, CDR1, CDR2, and CDR3 are indicated in brackets and are underlined. FR1 to FR4 are positioned at the sites indicated by lower-case letters on both sides of each underlined part.
H-chain V region, RV_{H}f (SEQ ID NO: 1):
qvqlqesgpglvrpsqtlsltctvsgysit [SDHAWS] wvrqppgrglewig [YIS-YSGITTYNPSLKS] rvtmlrdtsknqfslrlssvtaadtavyycar [SLARTTAMDY] wgqgslvtvs
   L-chain V region, RV_{L}a (SEQ ID NO: 2):
   diqmtqspsslsasvgdrvtitc [RASQDISSYLN] wyqqkpgkapklliy [YTSRLHS] gvpsrfsgsgsgtdftftisslqpediatyyc [QQGNTLPYT] fgqgtkveikr
The present invention also relates to polynucleotides that encode these modified antibodies. More specifically, the present invention provides polynucleotides encoding modified antibodies with an scFv connected to a human immunoglobulin IgG Fc region, in which the scFv comprises anti-IL6 receptor antibody H-chain and L-chain V regions, and CDRs comprising the following respective amino acid sequences incorporated into a human immunoglobulin framework:
   CDR1 in H-chain V region: SDHAWS (SEQ ID NO: 3)
   CDR2 in H-chain V region: YISYSGITTYNPSLKS (SEQ ID NO: 4)
   CDR3 in H-chain V region: SLARTTAMDY (SEQ ID NO: 5)
   CDR1 in L-chain V region: RASQDISSYLN (SEQ ID NO: 6)
   CDR2 in L-chain V region: YTSRLHS (SEQ ID NO: 7)
   CDR3 in L-chain V region: QQGNTLPYT (SEQ ID NO: 8)

Preferred structures constituting the modified antibodies of the present invention have been previously described. Nucleotide sequences that encode the amino acid sequences that provide the aforementioned structure can be designed based on these amino acid sequences. Alternatively, polynucleotides of the present invention can be constructed by modifying, according to an objective, an immunoglobulin-encoding gene obtained from antibody-producing cells. Specific methods for modification are as described above.

An example of a preferred polynucleotide of the present invention is a polynucleotide encoding a modified antibody comprising a polypeptide with the following structure:
H₂N-[H-chain V region]-[linker]-[L-chain V region]-[Fc]-COOH

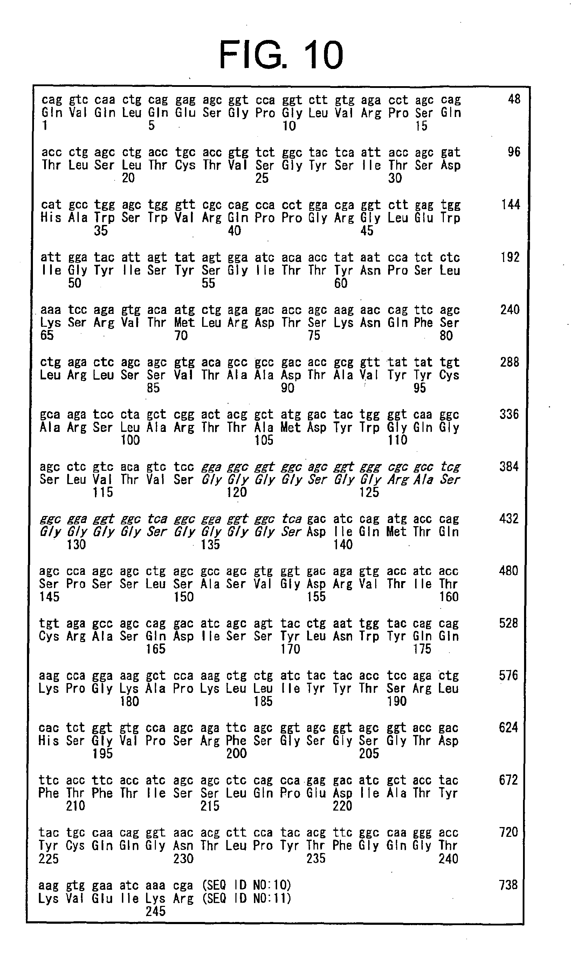

In the above-mentioned polypeptides, for example, the [H-chain V region] preferably comprises the amino acid sequence of SEQ ID NO: 1 (H-chain variable region). An example of the [L-chain V region] is the amino acid sequence of SEQ ID NO: 2. Furthermore, an example of a preferred amino acid sequence of a linker is the amino acid sequence of SEQ ID NO: 9. An example of a polynucleotide encoding a single chain-polypeptide with such a structure is a polynucleotide comprising the nucleotide sequence of SEQ ID NO: 10. The amino acid sequence of the scFv encoded by SEQ ID NO: 10 is shown in SEQ ID NO: 11. The linker regions in SEQ ID NO: 10 and SEQ ID NO: 11 are at positions 355-414 and positions 119-138, respectively. The linker regions are indicated in Fig. 10 using italics. Therefore, amino acid sequences corresponding to positions 1-118, 119-138, and 139-246 of SEQ ID NO: 11 are SEQ ID NO: 1, SEQ ID NO: 9, and SEQ ID NO: 2, respectively.

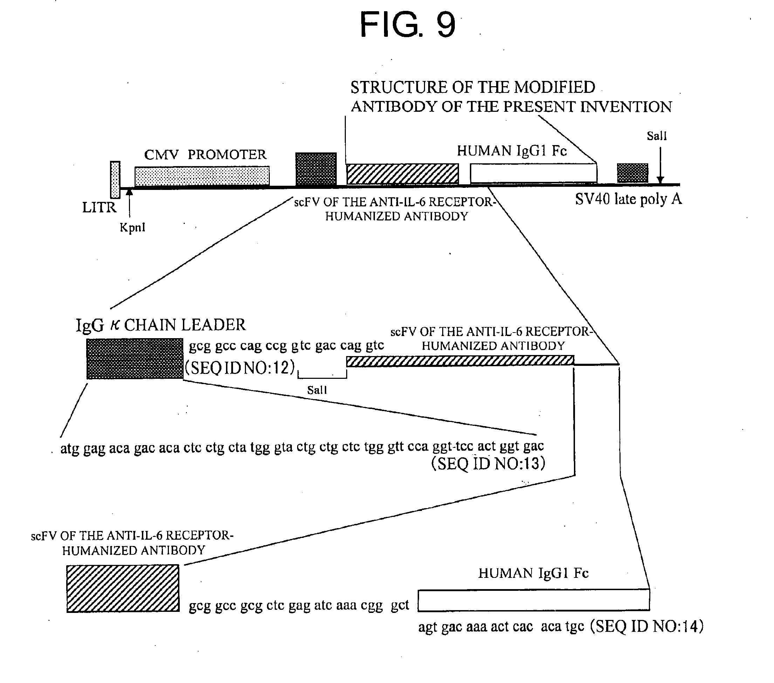

The polynucleotides of the present invention can be used to express the modified antibodies by using suitable expression vectors. To secrete the polynucleotide expression products outside of the cells, a polynucleotide encoding a leader sequence (secretion signal) can be added. A leader sequence is usually added to the N terminus of the [H-chain V region]. For example, the leader sequence of the human IgGκ chain shown in Fig. 9 can be used as a leader sequence. Any vectors that can express an immunoglobulin or its modified form can be used as the vectors.

Various vectors that can express immunoglobulins by using animal cells or microorganism cells as hosts are known ("Expression of engineered antibodies and antibody fragments in microorganisms", Methods Enzymol. 1989;178:476-96; and "Single chain antibody variable regions", Trends Biotechnol. 1991 Apr;9(4):132-7). Some known vectors include a leader sequence. When using such vectors, expression vectors that secrete the desired modified antibodies in animal cells can be constructed by connecting the polynucleotides of the present invention to the leader sequences of such vectors. Animal cells that can be used to express immunoglobulins are, for example, COS cells, CHO cells, or tumor cells such as malignant pleural mesothelioma cells.

Promoters ordinarily used for expression in animal cells can be used to express the polynucletoides that encode the modified antibodies of the present invention in these animal cells. For example, the use of the human cytomegalovirus (HCMV) immediate-early promoter is preferred. Examples of expression vectors comprising an HCMV promoter include HCMV-VH-HCγ1, HCMV-VL-HCK, HCMV-12h-gγ1, HCMV-12κ-gκ, and such, which are derived from pSV2neo.

An additional example of a promoter that can be used to express the modified antibodies of the present invention in animal cells is the human elongation factor-1α (HEF-1α) promoter. Expression vectors carrying this promoter include HEF-12h-gγ1 and HEF-12k-gκ, as well as HEF-VH-gγ1 and HEF-VL-gκ.

For gene amplification in the host cell system, expression vectors can further contain the dhfr gene. Expression vectors that carry the dhfr gene are, for example, DHFR-ΔE-PMh-gγ1, DHFR-ΔE-RVh-PM1-f, and the like.

The present invention also provides vectors comprising the polynucleotides of the present invention. Alternatively, the present invention relates to transfectants harboring the polynucleotides of the present invention or vectors comprising such polynucleotides in an expressible manner. Modified antibodies of the present invention can be produced by culturing the transfectants based on the present invention and recovering the modified antibodies of the present invention accumulated in the culture. The transfectants of the present invention can also be administered to patients. When the transfectants are to be administered to patients, the transfectant host cells are preferably human cells. More preferably, the cells are those of the patients who are to be administered the transfectants themselves. For example, peripheral blood lymphocytes can be used as such cells.

Mouse monoclonal antibody PM-1 has already been proven to be useful as a human IL-6 receptor inhibitor. Therefore, the modified antibodies of the present invention which have the antigen binding characteristics of this antibody are effective as IL-6 receptor inhibitors. IL-6 receptor inhibitors are also known to have therapeutic effects against various diseases. For example, by inhibiting the IL-6 receptor, therapeutic effects can be expected for all the following diseases:
chronic rheumatoid arthritis (Patent Document 2);
plasmacytosis, hyperimmunoglobulinemia, anemia, nephritis, cachexia, rheumatism,
Castleman's disease, and mesangioproliferative nephritis (Patent Document 3);
sensitized T cell-mediated diseases, such as multiple sclerosis, uveitis, chronic thyroiditis, delayed hypersensitivity, contact dermatitis, and atopic dermatitis (Patent Document 4);
systemic lupus erythematosus (Patent Document 4);
Crohn's disease (Patent Document 5);
pancreatitis (Patent Document 6);
psoriasis (Patent Document 7); and
childhood chronic arthritis (Patent Document 8).

Therefore, the modified antibodies of the present invention are useful as therapeutic agents for these diseases. Modified antibodies of the present invention can be made into pharmaceutical compositions by mixing with pharmaceutically acceptable carriers. More specifically, the present invention relates to the production of pharmaceutical compositions, where production comprises the step of mixing such modified antibodies with pharmaceutically acceptable carriers. The present invention also relates to uses of the modified antibodies of the present invention for the production of pharmaceutical compositions for treating such diseases.

Furthermore, the present invention provides interleukin-6 receptor inhibitors which comprise as active ingredients any of the components selected from the group consisting of modified antibodies of the present invention, polynucleotides encoding such antibodies, vectors harboring such polynucleotides in an expressible manner, and cells producing modified antibodies of the present invention. More specifically, the present invention relates to methods for inhibiting interleukin-6 receptor, where the methods comprise the step of administering to a patient any of the components selected from the group consisting of modified antibodies of the present invention, polynucleotides encoding such antibodies, vectors harboring such polynucleotides in an expressible manner, and cells producing the modified antibodies of the present invention.

The present invention also relates to uses of any of the components selected from the group consisting of modified antibodies of the present invention, polynucleotides encoding such antibodies, vectors harboring such polynucleotides in an expressible manner, and cells producing the modified antibodies of the present invention, in the production of pharmaceutical compositions for treating these diseases. In addition, the present invention relates to the use of any of the components selected from the group consisting of modified antibodies of the present invention, polynucleotides encoding such antibodies, vectors harboring such polynucleotides in an expressible manner, and cells producing the modified antibodies of the present invention in the treatment of these diseases.

Pharmaceutical compositions used for therapeutic or preventive purposes, which comprise modified antibodies of the present invention as active ingredients, can be formulated by blending, as necessary, with suitable pharmaceutically acceptable carriers, media, or such that do not react with the antibodies. For example, sterilized water, physiological saline solution, stabilizers, excipients, antioxidants (such as ascorbic acid), buffers (such as phosphate, citrate, and other organic acids), antiseptics, surfactants (such as PEG and Tween), chelating agents (such as EDTA), and binders may be used. They may also comprise other low-molecular-weight polypeptides, proteins such as serum albumin, gelatin, and immunoglobulins; amino acids such as glycine, glutamine, asparagine, arginine, and lysine; carbohydrates such as polysaccharides and monosaccharides; and sugar alcohols such as mannitol and sorbitol. When preparing aqueous solutions for injection, physiological saline solutions and isotonic solutions containing glucose, and other adjuvants, such as D-sorbitol, D-mannnose, D-mannitol, and sodium chloride, may be used in combination with suitable solubilizers, such as alcohols (such as ethanol), polyalcohols (such as propylene glycols and PEGs), and non-ionic surfactants (for example, Polysorbate 80 and HCO-50) and the like.

If necessary, modified antibodies of the present invention may be encapsulated in microcapsules (microcapsules made of hydroxymethylcellulose, gelatin, poly(methylmetacrylate), and such), or made into colloidal drug delivery systems (such as liposomes, albumin microspheres, microemulsion, nanoparticles, and nanocapsules) (see for example, "Remington's Pharmaceutical Science 16th edition", Oslo Ed. (1980)). Methods for preparing the pharmaceutical agents as controlled-release pharmaceutical agents are also well known, and such methods may be applied to the modified antibodies of the present invention (Langer et al., J. Biomed. Mater. Res. 15: 167-277 (1981); Langer, Chem. Tech. 12: 98-105 (1982); U.S. Patent No. 3,773,919; European Patent (EP) Patent Application No. 58,481; Sidman et al., Biopolymers 22: 547-556 (1983); EP 133,988).

The doses of the pharmaceutical compositions of the present invention are determined appropriately by considering the type of dosage form, method of administration, patient age and body weight, symptoms of the patient, or degree of disease progress, and doses are ultimately decided by physicians. However, generally, the daily dose for an adult is 0.1-2000 mg, and is administered orally at once or in several portions. The dose is more preferably 1-1000 mg/day, more preferably 50-500 mg/day, and most preferably 100-300 mg/day. Doses may vary depending on the patient body weight and age, and the method of administration, but those skilled in the art can appropriately select suitable doses. Preferably, the duration of administration is also appropriately determined depending on the patient's healing progress and such.

Based on the present invention, pharmaceutical packages are provided that comprise pharmaceutical compositions comprising any of the components selected from the group consisting of modified antibodies of the present invention, polynucleotides encoding such antibodies, vectors harboring such polynucleotides in an expressible manner, and cells producing the modified antibodies of the present invention, along with pharmaceutically acceptable carriers, and an instruction sheet indicating that these pharmaceutical compositions are to be used for treating the aforementioned diseases. Diseases for which the pharmaceutical agent is indicated, doses, administration schedules, contraindications, conditions for storage of the pharmaceutical compositions, expiration dates, or such may be indicated in the instruction sheet. The formation of pharmaceutical packages enables commercial distribution of the pharmaceutical compositions based on the present invention.

Gene therapy may also be performed by inserting genes encoding the modified antibodies of the present invention into gene therapy vectors. Besides direct administration using naked plasmids, the methods of administration include administration after packaging into liposomes and such, forming a variety of viral vectors such as retrovirus vectors, adenovirus vectors, vaccinia virus vectors, poxvirus vectors, adeno-associated virus vectors, and HVJ vectors and the like (see Adolph "Virus Genome Method" CRC Press, Florida (1996)), or coating on to carrier beads such as colloidal gold particles (WO 93/17706 and such). However, as long as the antibodies are expressed *in vivo* and their functions are exercised, there is no limitation to their method of administration.

Preferably, sufficient doses can be administered by suitable parenteral routes (such as injecting intravenously, intraperitoneally, subcutaneously, intradermally, into adipose tissues or mammary glands, inhalation, intramuscular injection, infusion, or gas-driven particle bombardment (using electron guns and such), or through the mucosa, for example, by nasal drops). Alternatively, genes encoding modified antibodies of the present invention may be administered *ex vivo* into blood cells, bone marrow cells, and such using liposome transfection, particle bombardment (U.S. Patent No. 4,945,050), or viral infection, and the cells may be reintroduced into animals.

Modified antibodies of the present invention are particularly advantageous when applied to gene therapy. Generally, antibody pharmaceuticals such as IgG are thought to be difficult to apply to gene therapies since they have multimeric structures. This is because it is currently difficult for expression products from gene therapy vectors to take the same multimeric structures as naturally derived antibodies. However, the modified antibodies of the present invention can be expressed as single-chain polypeptides. Therefore, even at the technical level of gene therapies currently being tested, a target protein (modified antibody) could be expressed in a form that has antibody activity. To mimic the multimeric structure of IgG, the formation of dimers is desirable. Dimers formed by the modified antibodies of the present invention are composed of two homodimers. Homodimers constructed using the same molecules can be reproduced much more easily than when dimers are formed using different polypeptides, such as H chain and L chains, using genetically modified organisms.

For example, for heterodimers, each of the subunits constituting a heterodimer must be present in their required quantities. One subunit expressed more than the other subunit cannot form a heterodimer without its partner subunit. On the other hand, with homodimers, the expressed subunit can form homodimers almost to completion. Therefore, even under conditions in which the expression level is not highly controlled, the target homodimers can be efficiently produced. For this reason, the modified antibodies of the present invention are preferred for gene therapies.

More specifically, the present invention provides gene therapy vectors that harbor polynucleotides encoding modified antibodies of the present invention such that they can be expressed in human cells. Gene therapy vectors of the present invention harbor polynucleotides encoding the aforementioned modified antibodies and can express the modified antibodies in human cells upon introduction into or infection of human cells.

Introduction of a gene therapy vector into human cells includes introduction of a gene therapy vector into human cells by a step of artificial transfection. More specifically, vectors can be introduced into human cells using transfection reagents such as Lipofectamine. Alternatively, gene therapy vectors that can autonomously infect human cells without the help of transfection reagents are also known. For example, viral vectors such as adenovirus vectors, adeno-associated virus vectors, retrovirus vectors, HIV vectors, vaccinia virus vectors, or Sendai virus (HVJ) vectors are vectors that have the ability to infect human cells. More specifically, adenovirus vectors such as pAdexlcw, retrovirus vectors such as pZIPneo, or such are known. DNAs encoding the modified antibodies of the present invention can be inserted into viral vectors by performing conventional genetic manipulations (Molecular Cloning, 5.61-5.63).

For example, adenovirus vectors that express modified antibodies based on the present invention can be constructed by manipulations such as the following: Most adenovirus vectors currently produced are based on serotype 5 or serotype 2. The E1 region of an adenovirus is a region essential for replication and growth, and by substituting this region with a desired gene, a non-proliferative adenovirus can be produced. Adenovirus vectors are produced using human kidney 293 cells, or such. Production of a desired vector requires production of a linear DNA that spans over 36 kb. As methods for producing such enormous DNAs, there is the method of transfecting a shuttle vector and an adenovirus backbone vector directly into host cells and then inducing homologous recombination inside the cells, and the method of using bacteria to produce an enormous DNA required for viral production. An example of the latter is a method that uses the AdEasy Adenoviral Vector System (Stratagene).

For secretion of the expressed modified antibodies to the outside of cells, modified antibodies ordinarily comprise a secretion signal. Any promoter having transcriptional activity in human cells can be used as a promoter that regulates expression of genes encoding a modified antibody. By combining a controllable promoter such as tet, the expression levels of the modified antibodies can be regulated using administration of tetracycline. The use of enhancers is preferred for maintaining the expression levels of the modified antibodies.

The gene therapy vectors of the present invention can be administered to humans using any methods. For example, cells to which a vector has been introduced *ex vivo* can be administered to patients. The cells are preferably those collected from the patients themselves. Cells collected from the patients can be grown by culturing them *ex vivo.* Cells easily collected from patients include blood cells. For example, peripheral blood lymphocytes are preferred as cells for introducing gene therapy vectors.

Alternatively, when gene therapy vectors have the ability to infect cells even *in vivo,* the gene therapy vectors can be administered directly to patients. More specifically, subcutaneous, blood, intrathoracic, intraperitoneal, intrathecal, intramuscular, intraarticular, transnasal, or transdermal administration is possible.

Elements necessary for the therapeutic methods that use these gene therapy vectors based on the present invention can be pre-combined to form kits. More specifically, the present invention provides kits for treating diseases caused by the action of interleukin-6, where the kits comprise the following elements:
(1) a vector harboring a polynucleotide encoding a modified antibody of the present invention such that it can be expressed in human cells;
(2) a vector transfection reagent; and
(3) a reagent for peripheral blood lymphocyte separation.

As the vector transfection reagent, Lipofectin, Lipofectamine (both are product names of Invitrogen: Registered trade mark), or such may be used. As the reagent for peripheral blood lymphocyte separation, antibodies that recognize markers for lymphocyte identification may be used. The antibodies can be coupled to fluorescent dyes or magnetic particles. Lymphocyte markers such as CD34 are well known. The kits of the present invention for gene therapy can additionally comprise instruction sheets. All prior art references cited herein are incorporated by reference into this description.

### Examples

### <Materials>

Human embryonic kidney 293 cells were purchased from American Type Culture Collection (Manassas, VA). The 293 cells were cultured in Dulbecco's modified Eagle's medium (DMEM) with 10% fetal calf serum (FCS). Lennert's lymphoma-derived T-cells (KT-3) (Shimizu S. et al., Blood 1988; 72:1826-1828) were maintained in RPMI1640 medium supplemented with 10% FCS and 4 ng/ml of recombinant IL-6 (Ajinomoto Co., Inc., Kawasaki, Japan). A recombinant soluble IL-6R (sIL-6R) was given by Chugai Pharmaceutical Co., Ltd (Roche Group, Shizuoka, Japan). Other supplements and cell culture media were purchased from Mediatech-Cellgro (Kansas City, MO). All restriction enzymes were purchased from New England Biolabs (Beverly, MA); T4 DNA ligase was purchased from Promega (Madison, WI); and Taq polymerase and PCR reagents were purchased from QIAGEN (Valencia, CA).

### [Example 1]

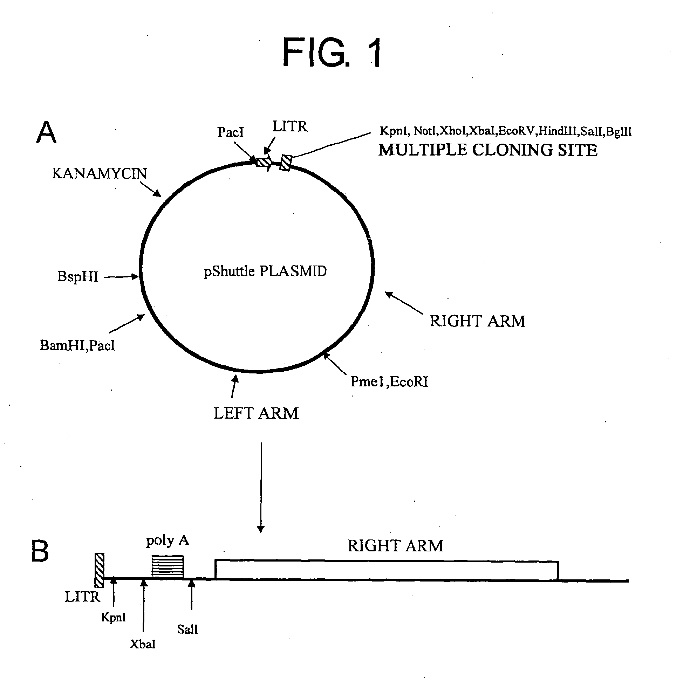

### (Figs. 1-3) Production of an IL-6 inhibitor (modified antibody)

The VH and VL nucleotide sequences were amplified by PCR from separate cDNAs encoding heavy and light chains of the humanized IL-6 receptor antibody (Chugai Pharmaceutical Co., Ltd). PCR primers for the first round of amplification were designed using canonical Fv boundary definition. In the second round, VH and VL gene fragments were connected using the overlap extension PCR method, and a DNA segment encoding a 20 amino acid linker (G₄S)(GGRAS)(G₄S)₂ was introduced. In addition, Nco I and Not I restriction sites were introduced at the 5' and 3' ends of the final scFv-coding DNA, respectively, to facilitate cloning into pOPE101 bacterial expression vector, and pOPE101/scFv of the anti-IL-6 receptor humanized antibody was generated.

To produce the scFv in a eukaryotic expression system, the DNA sequence encoding the scFv was PCR-amplified together with the c-Myc tag and six histidines (present in pOPE101), and Sna BI and Avr I were introduced to the 5' end and 3'end respectively, and then cloned to pPIC9 yeast expression plasmid (Invitrogen, Carlsbad, CA), generating pPIC9/scFv of the anti-IL-6 receptor humanized antibody. The Fc/anti-IL-6 receptor humanized antibody coding DNA was assembled as follows. The CH2 and CH3 domains (collectively called as Fc or fragment constant), the hinge of the human immunoglobulin γ-1 gene, was PCR-amplified from the cDNA of the heavy chain of the humanized IL-6 receptor antibody. Then a substitution of a single amino acid from cysteine to serine was introduced in the hinge region. This cysteine residue normally forms a disulfide bond with the light chain.

The Fc gene fragment was cloned into pSecTag2a vector (Invitrogen) in frame with the murine Igκ-chain V-J2-C signal peptide sequence. A gene of the scFv of the anti-IL-6 receptor humanized antibody was PCR-amplified and cloned in frame with the leader/Fc, generating a pSecTag/Fc/scFv of the anti-IL-6 receptor humanized antibody. Finally the whole expression cassette including a CMV promoter and BGH polyadenylation signal was re-cloned into pShuttle plasmid (Stratagene, La Jola, CA), generating pShuttle/CMV/Fc/scFv of the anti-IL-6 receptor humanized antibody. The positions of each of the elements constituting the construct and the nucleotide sequences that form connections between the elements are shown in Fig. 9.

(Fig. 1B) An SV40 late poly A signal derived from a pGL3 basic Vector (Promega) was inserted into the multiple cloning site of pShuttle plasmid (Stratagene, La Jola, CA).

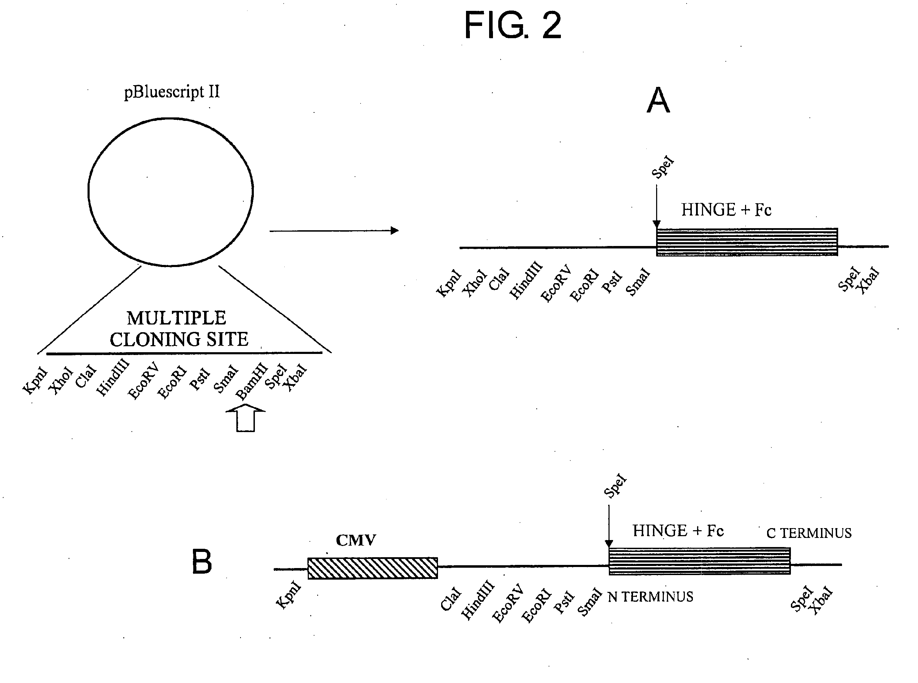

(Fig. 2A) The C terminus of the hinge region of the H chain of human immunoglobulin IgG1 was subcloned into the BamHI site of pBluescript II. A SpeI restriction site was introduced at the N terminus of the hinge region.

(Fig. 2B) In the above-mentioned plasmid, a cytomegalovirus (CMV) enhancer/promoter region was inserted into the KpnI-XhoI site of pBluescript II.

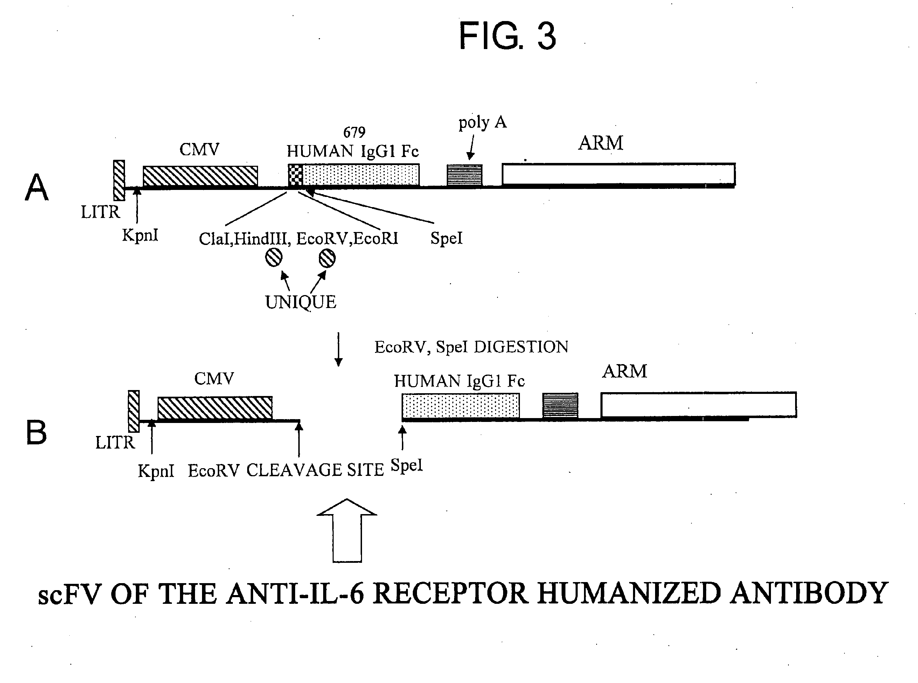

(Fig. 3A) The CMV-multiple cloning site-hinge-Fc fragment was cut from the above-mentioned plasmid and inserted into the KpnI-XbaI site of the plasmid of Fig. 1B. EcoRV and SpeI sites are unique sites in this plasmid, and incorporation of another gene into these sites allows production of a protein in which that gene and Fc are fused. Cutting the scFv region from the above-mentioned pShuttle/CMV/Fc/scFv of the anti-IL-6 receptor humanized antibody and then inserting it into the EcoRV-SpeI site completed the production of the IL-6 inhibitor (modified antibody) expression shuttle vector.

To generate a recombinant adenovirus genome, AdEasy Adenoviral Vector System (Stratagene) was used in accordance with the instruction manual. The IL-6 inhibitor expression shuttle vector was co-transfected with pAdEasy-1 plasmid in BJ5183 *E. coli* bacteria to achieve homologous recombination. The viral genome was recovered from the cells and the presence of the IL-6 inhibitor DNA was confirmed using a restriction digest and PCR with insert-specific primers. The recombinant Ad virus genome was transfected to 293 cells, and recombinant Ad5/IL-6 inhibitor was rescued and mass-produced, and then purified using cesium chloride. The viral particle titer was determined spectrophotometrically by the method of Maizel *et al.,* using a conversion coefficient of 1.1 × 10¹² viral particles (v.p.) per one absorbance unit at 260 nm.

To mass-produce the IL-6 inhibitor, recombinant Ad5/IL-6 inhibitor was used as an expression system. The 293 cells in ten T75 flasks were infected with the Ad5/IL-6 inhibitor at a multiplicity of infection (MOI) of 100 v.p./cell. Infection was carried out for one hour at 37°C, and then the medium was replaced. Three days after the infection, the culture supernatant was collected and the proteins were precipitated by addition of an equal volume of cold-saturated ammonium sulfate. The precipitate was collected by centrifugation, dissolved in 1/20th of the original medium volume of phosphate-buffered saline (PBS) and then dialyzed against PBS. The recombinant IL-6 inhibitor protein was purified from the dialyzed protein solution by affinity chromatography using Protein A Antibody Purification Kit purchased from Sigma (St. Louis, MO), according to the manufacturer's instructions. Protein concentration was determined using the Bradford protein assay (Bio-Rad), with bovine γ globulin as a standard.

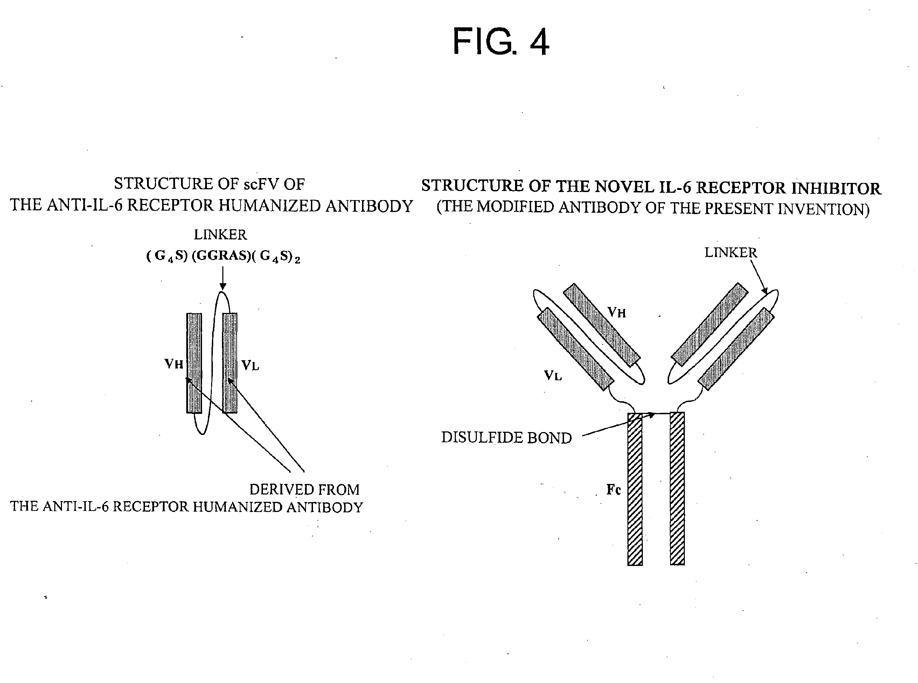

(Fig. 4) First, the present inventors used the H and L chains of the anti-IL-6 receptor humanized antibody to generate a single chain fragment variant (scFv of the anti-IL-6 receptor humanized antibody). This scFv was fused to human immunoglobulin IgG Fc regions to generate dimers and establish new receptor inhibitors for IL-6 (the modified antibodies of the present invention).

Next, a gene of a modified antibody of the present invention was inserted into a non-proliferative adenovirus, and a vector expressing the modified antibody of the present invention downstream of a cytomegalovirus promoter was generated. A variety of human cells were infected with this adenovirus (Ad CMV) that expresses modified antibodies of the present invention, the supernatants of these cultures were collected, and the modified antibodies of the present invention were purified using Protein A columns.

### [Example 2]

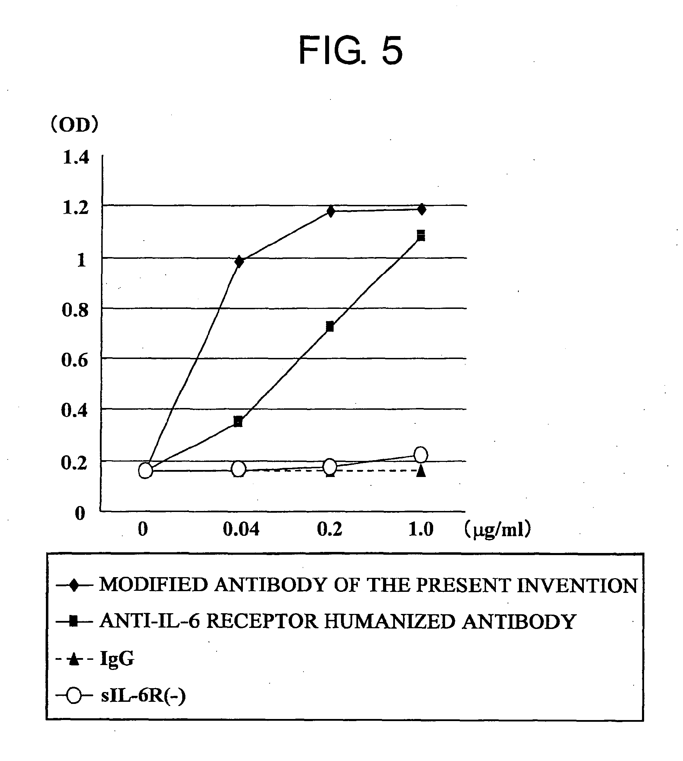

### (Fig. 5) Binding assays

Affinity of a modified antibody of the present invention to the IL-6 receptor was examined. First, a 96-well ELISA plate was coated with MT-18, which recognizes the IL-6 receptor. The recognition site for IL-6 receptor of MT-18 is different from the anti-IL-6 receptor humanized antibody, and it has been proven that MT-18 does not compete with the anti-IL-6 receptor humanized antibody. The concentration of the MT-18 coating was 5 µg/mL; 100 µL of this solution was added to each well and left to stand overnight. This was followed by washing with tris buffered saline solution (TBS), and blocking with 6% bovine serum albumin. After blocking, soluble IL-6 receptor (sIL-6R) was reacted and fixed to the ELISA plate. 100 µL of an sIL-6R solution (concentration: 100ng/mL) was used for reaction in each well. Thereafter, various concentrations of the modified antibody of the present invention, anti-IL-6 receptor humanized antibody, and control IgG were reacted. HRP conjugated anti-Fc fragment F(ab')2 (Dako) was used as a secondary antibody. OPD peroxidase substrate (Sigma) was used for coloring. In Fig. 4, the horizontal axis shows the reacted concentrations of the modified antibody of the present invention, anti-IL-6 receptor humanized antibody, and control IgG. This shows that the ability of the modified antibody of the present invention to bind to the IL-6 receptor compares favorably with that of the anti-IL-6 receptor humanized antibody.

### [Example 3]

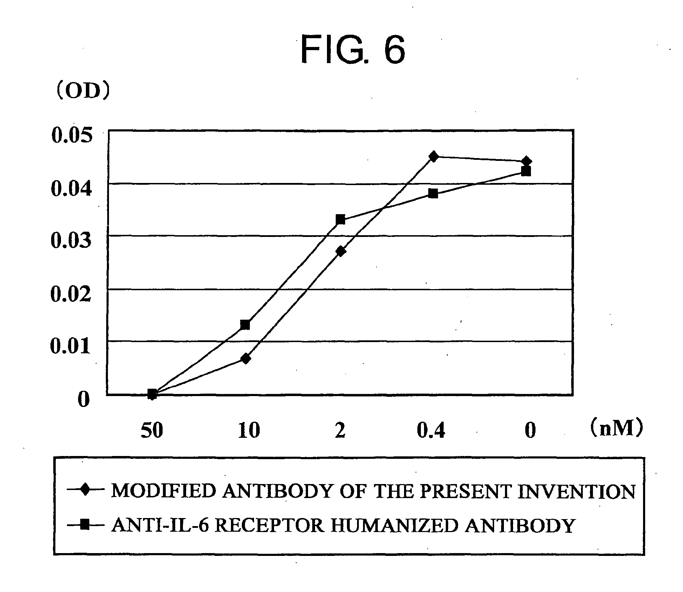

### (Fig. 6) Inhibition Assays

The present inventors then examined whether a modified antibody of the present invention can competitively inhibit binding of IL-6 to IL-6 receptor. sIL-6R was fixed to ELISA plates as described above. The results of the competition of biotinylated IL-6 at 10 ng/mL with various concentrations of a modified antibody of the present invention or of anti-IL-6 receptor humanized antibody were that the ability of the modified antibody of the present invention to inhibit binding to the IL-6 receptor was comparable to that of the anti-IL-6 receptor humanized antibody.

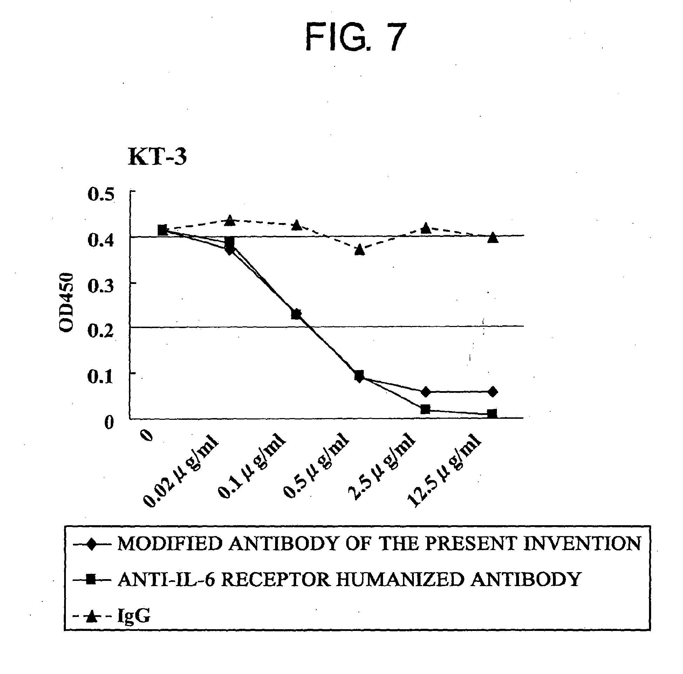

(Fig. 7) Using lymphoma KT-3, which shows IL-6-dependent growth, the ability of a modified antibody of the present invention to actually cytologically block IL-6 stimulus was examined. The concentration of KT-3 cells was 10,000 cells/well, and 96 wells were used. Various concentrations of the modified antibody of the present invention, anti-IL-6 receptor humanized antibody, or control IgG were administered to KT-3 cultured at an IL-6 concentration of 500 pg/mL. After culturing for five days, MTS assays were performed. The results are shown in Fig. 7. The KT-3 growth suppressing effect was also comparable to that of anti-IL-6 receptor humanized antibody.

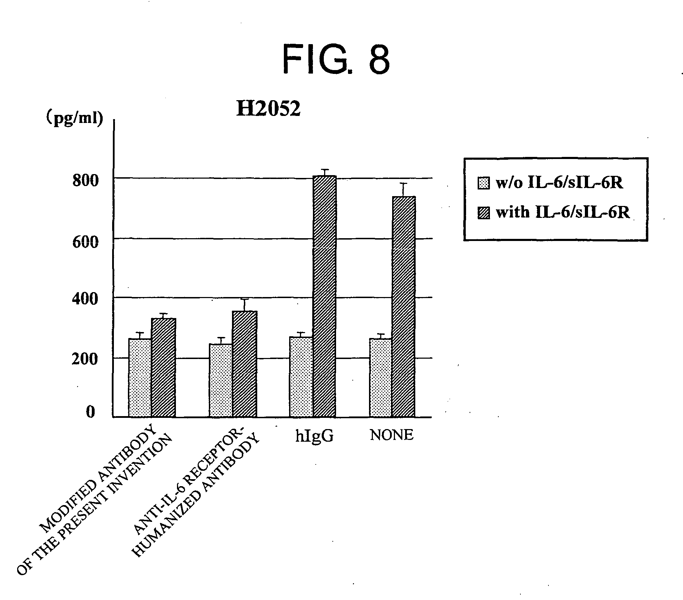

(Fig. 8) The malignant pleural mesothelioma, H2052, has been known to increase VEGF production due to IL-6 and sIL-6R stimuli. This H2052 was used to examine whether a modified antibody of the present invention can suppress the induction of VEGF production caused by IL-6 stimulation. Culturing of the H2052 malignant mesothelioma cells was initiated at a cell concentration of 50,000 cells/well using a 20-well plate. After letting this stand overnight, the media was replaced with that including 5 nM of a modified antibody of the present invention, an anti-IL-6 receptor humanized antibody, a human IgG, or a control. Stimulation with IL-6 (10 ng/mL) + sIL-6R (100 ng/mL) was initiated, or there was no stimulation. The experiments were performed in triplicate. After culturing for 48 hours, the concentration of VEGF in the culture supernatant was measured, and the production levels were examined. The results are shown in Fig. 8. In this system also, the modified antibody of the present invention showed an equivalent suppressive ability as the original anti-IL-6 receptor humanized antibody.

### Industrial Applicability

The present invention provides modified antibodies useful as IL-6 inhibitors. The modified antibodies of the present invention can be expressed as single-chain polypeptides. Therefore, the modified antibodies of the present invention are particularly useful in gene therapy. Specifically, the modified antibodies, which are the active ingredients, can be expressed *in vivo* in the patient by introducing polynucleotides encoding the modified antibodies of the present invention into patients. Since the modified antibodies are single-chain polypeptides, they can be easily expressed *in vivo,* and also allow efficient production of homodimers that mimic the structure of naturally derived IgGs. For example, modified antibodies actually produced based on the present invention were found to have anticancer activity equivalent to that of naturally derived IgGs.

## Claims

1. A modified antibody with an scFv connected to a human immunoglobulin IgG Fc region, wherein said scFv comprises an anti-IL6 receptor antibody H-chain and L-chain V region, and CDRs comprising of the following amino acid sequences incorporated into a human immunoglobulin framework:
CDR1 in H-chain V region: SDHAWS (SEQ ID NO: 3)
CDR2 in H-chain V region: YISYSGITTYNPSLKS (SEQ ID NO: 4)
CDR3 in H-chain V region: SLARTTAMDY (SEQ ID NO: 5)
CDR1 in L-chain V region: RASQDISSYLN (SEQ ID NO: 6)
CDR2 in L-chain V region: YTSRLHS (SEQ ID NO: 7)
CDR3 in L-chain V region: QQGNTLPYT (SEQ ID NO: 8).

2. The modified antibody of claim 1, wherein the H-chain V region consists of the amino acid sequence of QVQLQESGPGLVRPSQTLSLTCTVSGYSITSDHAWSWVRQPPGRGLEWIG YIS-YSGITTYNPSLKS RVTMLRDTSKNQFSLRLSSVTAADTAVYYCAR SLARTTAMDY WGQGSLVTVS (SEQ ID NO: 1).

3. The modified antibody of claim 1, wherein the L-chain V region consists of the amino acid sequence of DIQMTQSPSSLSASVGDRVTITC RASQDISSYLN WYQQKPGKAPKLLIY YTSRLHS GVPSRFSGSGSGTDFTFTISSLQPEDIATYYC QQGNTLPYT FGQGTKVEIKR (SEQ ID NO: 2).

4. The modified antibody of claim 1, wherein the scFv is connected to the human immunoglobulin IgG Fc region at the C terminus of the L-chain V region.

5. The modified antibody of claim 4, wherein the scFv comprises an H-chain V region, a linker, and an L-chain V region connected in that order from the N terminus.

6. The modified antibody of claim 5, wherein the amino acid sequence of the linker is GGGGSGGRASGGGGSGGGGS (SEQ ID NO: 9).

7. The modified antibody of claim 1, which is a dimer connected via an SS bond.

8. A pharmaceutical composition comprising the modified antibody of any one of claims 1 to 7 and a pharmaceutically acceptable carrier.

9. A method for manufacturing a pharmaceutical composition comprising the step of combining the modified antibody of any one of claims 1 to 7 and a pharmaceutically acceptable carrier.

10. A polynucleotide encoding a modified antibody which comprises an scFv connected to a human immunoglobulin IgG Fc region, wherein said scFv comprises an anti-IL-6 receptor antibody H-chain and L-chain V region, and CDRs comprising of the following amino acid sequences incorporated into a human immunoglobulin framework:
CDR1 in H-chain V region: SDHAWS (SEQ ID NO: 3)
CDR2 in H-chain V region: YISYSGITTYNPSLKS (SEQ ID NO: 4)
CDR3 in H-chain V region: SLARTTAMDY (SEQ ID NO: 5)
CDR1 in L-chain V region: RASQDISSYLN (SEQ ID NO: 6)
CDR2 in L-chain V region: YTSRLHS (SEQ ID NO: 7)
CDR3 in L-chain V region: QQGNTLPYT (SEQ ID NO: 8).

11. The polynucleotide of claim 10, wherein the H-chain V region consists of the amino acid sequence of QVQLQESGPGLVRPSQTLSLTCTVSGYSIT SDHAWS WVRQPPGRGLEWIG YIS-YSGITTYNPSLKS RVTMLRDTSKNQFSLRLSSVTAADTAVYYCAR SLARTTAMDY WGQGSLVTVS (SEQ ID NO: 1).

12. The polynucleotide of claim 10, wherein the L-chain V region consists of the amino acid sequence of DIQMTQSPSSLSASVGDRVTITC RASQDISSYLN WYQQKPGKAPKLLIY YTSRLHS GVPSRFSGSGSGTDFTFTISSLQPEDIATYYC QQGNTLPYT FGQGTKVEIKR (SEQ ID NO: 2).

13. The polynucleotide of claim 10, wherein the scFv is connected to a human immunoglobulin IgG Fc region at the C terminus of an L-chain V region.

14. The polynucleotide of claim 13, wherein the scFv comprises an H-chain V region, a linker, and an L-chain V region connected in that order from the N terminus.

15. The polynucleotide of claim 14, wherein the amino acid sequence of the linker is GGGGSGGRASGGGGSGGGGS (SEQ ID NO: 9).

16. A vector comprising the polynucleotide of any one of claims 10 to 15.

17. A transformant harboring the polynucleotide of any one of claims 10 to 15 or a vector comprising said polynucleotide in an expressible manner.

18. The transformant of claim 17, wherein the transformant is derived from a human cell.

19. A method for manufacturing a modified antibody comprising the steps of culturing the transformant of claim 17 and recovering a modified antibody accumulated in culture.

20. An IL-6 receptor inhibitor comprising as an active ingredient a component selected from the group consisting of the modified antibody of any one of claims 1 to 7; the polynucleotide of any one of claims 10 to 15; a vector carrying the polynucleotide of any one of claims 10 to 15 in an expressible manner; and a cell producing the modified antibody of any one of claims 1 to 7.

21. A method for inhibiting IL-6 receptor comprising the step of administering any components selected from the group consisting of the modified antibody of any one of claims 1 to 7; the polynucleotide of any one of claims 10 to 15; a vector carrying the polynucleotide of any one of claims 10 to 15 in an expressible manner; and a cell producing the modified antibody of any one of claims 1 to 7.

22. A gene therapy vector comprising the polynucleotide of any one of claims 10 to 15 such that it can be expressed in a human cell.

23. A method for treating a disease caused by an action of IL-6, wherein the method comprises the steps of:
(1) introducing into a cell a vector carrying the polynucleotide of any one of claims 10 to 15 such that it can be expressed in a human cell; and
(2) administering the vector-introduced cell to a patient.

24. The method of claim 23, wherein the cell is obtained from a patient to be treated.

25. The method of claim 24, wherein the cell is a peripheral blood lymphocyte.

26. A therapeutic kit for a disease caused by an action of IL-6, wherein the kit comprises the following elements:
(1) a vector carrying the polynucleotide of any one of claims 10 to 15 such that it can be expressed in a human cell;
(2) a reagent for vector introduction; and
(3) a reagent for peripheral blood lymphocyte isolation.
